**Europäisches Patentamt**

**European Patent Office**

**Office européen des brevets**

(11) Veröffentlichungsnummer: **0 436 149 A1**

# EUROPÄISCHE PATENTANMELDUNG

(21) Anmeldenummer: **90123708.1**

(22) Anmeldetag: **10.12.90**

(51) Int. Cl.5: **A61B 17/32, A61B 17/22**

(30) Priorität: **22.12.89 DE 3942589**

(43) Veröffentlichungstag der Anmeldung:
**10.07.91 Patentblatt 91/28**

(84) Benannte Vertragsstaaten:
**DE FR GB IT**

(71) Anmelder: **OLYMPUS OPTICAL CO. (EUROPA) GMBH**
**Wendenstrasse 14-16**
**W-2000 Hamburg 1(DE)**

(72) Erfinder: **Stierli, Peter, Dr.**
**Talbachstrasse 147**
**CH-5722 Gränichen(CH)**

(74) Vertreter: **Schaefer, Konrad**
**Gehölzweg 20**
**W-2000 Hamburg 70(DE)**

(54) **Venenklappenschneidmesser.**

(57) Ein Messer zum Aufschneiden von Venenklappen (3) mit einem langen, dünnen, einen Kanal aufweisenden Schaft (4) und einer an dessen distalem Ende radial abstehend angeordneten Klinge (5) mit rückwärts gerichteter Schneide (6) ist dadurch gekennzeichnet, daß der Kanal als am distalen Ende mit gekrümmtem Übergang seitlich abgewinkelter Arbeitskanal ausgebildet ist.

## VENENKLAPPENSCHNEIDMESSER

Die Erfindung betrifft ein Messer der im Oberbegriff des Anspruches 1 genannten Art.

Derartige Messer werden in der Gefäßchirurgie benötigt, und zwar insbesondere bei der Behandlung von Durchblutungsstörungen im Bein (Raucherbein) durch Schaffung eines natürlichen Arterienbypass. Bei funktionsunfähigen Arterien können als Bypass Kunststoffschläuche verwendet werden oder auch im Körper vorhandene Venen, die als Arterien verwendet werden.

Wenn eine Vene als Arterie eingesetzt wird, aber an Ort und Stelle liegenbleibt, wird sie in der falschen Richtung durchströmt. Die in der Vene vorhandenen Venenklappen, die einfache Rückschlagventile darstellen, würden dann den Blutfluß verhindern. Sie müssen also funktionsunfähig gemacht werden. Dazu dienen die Messer der eingangs genannten Art.

Ein passendes Venenstück wird an den Enden abgeklemmt und durch Einstiche zugänglich gemacht. Das Messer wird vom körperfernen Ende des Venenstückes her in der ursprünglichen Blutstromrichtung durch die ganze Länge des Venenstückes nichtschneidend geführt. Dabei geht das Messer relativ leicht in Öffnungsrichtung durch die Venenklappen. Sodann wird das Messer wieder zurückgezogen und schneidet erst dann mit seiner rückwärtig scharfen Schneide durch die Venenklappen und macht diese funktionsunfähig.

Um die Klappen beim Zurückziehen möglichst geschlossen zu halten, wird mit Spülflüssigkeit - in der Regel physiologische Kochsalzlösung - zur Erzielung eines Schließdruckes gespült. Diese Spülflüssigkeit kann durch Kanäle über das Messer oder ein gesondert eingeführtes Endoskop mit Spülkanal zugeführt werden.

Es sind verschiedene Venenklappenmesser dieser Art in WO 89/07 914 und WO 89/06 936 beschrieben.

Da die zu behandelnden Venen Seitenäste bzw. Abzweigvenen aufweisen, ist es für deren späteren Betrieb als Arterie wesentlich, diese Seitenäste zu verschließen um spätere Kurzschlüsse zwischen dem arteriellen und venösen Blutsystem zu verhindern. Nach dem Stand der Technik müssen zu diesem Zweck die Abzweigvenenvenen von außen zugängig gemacht und bearbeitet werden.

Die Aufgabe der vorliegenden Erfindung ist es daher, ein Messer der eingangs genannten Art zu schaffen, mit dessen Hilfe auch das Behandeln und Verschließen von Zweigvenen ermöglicht wird.

Diese Aufgabe wird erfindungsgemäß mit den Merkmalen des Kennzeichnungsteils des Anspruches 1 gelöst.

Das erfindungsgemäße Messer weist in seinem Schaft einen Arbeitskanal auf. Bei sehr dünnem Schaft könnte der Arbeitskanal auch durch einen parallel verlegten Schlauch ausgebildet sein. Durch den gekrümmten Übergang vom achsialen zum seitlich abgewinkelten distalen vorzugsweise starren Endteil des Arbeitskanals ist es möglich, Sonden einzuführen, mit denen Eingriffe nunmehr an der Venenwand und insbesondere in seitlichen Venenzuflüssen durchgeführt werden können. Die seitlichen Venenzuflüsse können z.B. mit geeigneten Mitteln - auch unter optischer Kontrolle - verstopft werden. Die optische Kontrolle wird durch ein vom anderen Ende der Vene eingeführtes Endoskop ermöglicht. Das Einschneiden der Venenklappen durch Zurückziehen des Messers kann ebenfalls unter optischer Kontrolle erfolgen.

Vorteilhaft sind dabei die Merkmale des Anspruchs 2 vorgesehen. Ist der Schaft selbst als Rohr ausgebildet, so ergibt sich zusätzlich der Vorteil einer sehr festen und steifen Schaftkonstruktion, die sowohl starr in Metall ausführbar ist als auch als flexibler Schlauch.

Vorteilhaft sind auch die Merkmale des Anspruchs 3 vorgesehen. Wird die Austrittsöffnung des Arbeitskanals am freien Ende der Klinge angeordnet, kann der distale Teil des Arbeitskanals besonders raumsparend im abgerundeten Teil der Klinge untergebracht werden.

Weiterhin vorteilhaft sind die Merkmale des Anspruchs 4 vorgesehen. Durch gleichzeitige Verwendung des Arbeitskanals als Spülkanal kann auf die Verwendung eines Endoskopes mit Spülkanal verzichtet werden. Der Querschnitt des Endoskopes kann daher kleiner gewählt werden. Ein Arbeiten in sehr engen Venen unter optischer Kontrolle ist daher noch möglich.

Dabei sind vorteilhaft die Merkmale des Anspruchs 5 vorgesehen. Durch zusätzliche Ausströmöffnungen in Richtung Endoskop können die Spül- und insbesondere die Sichtverhältnisse weiter verbessert werden.

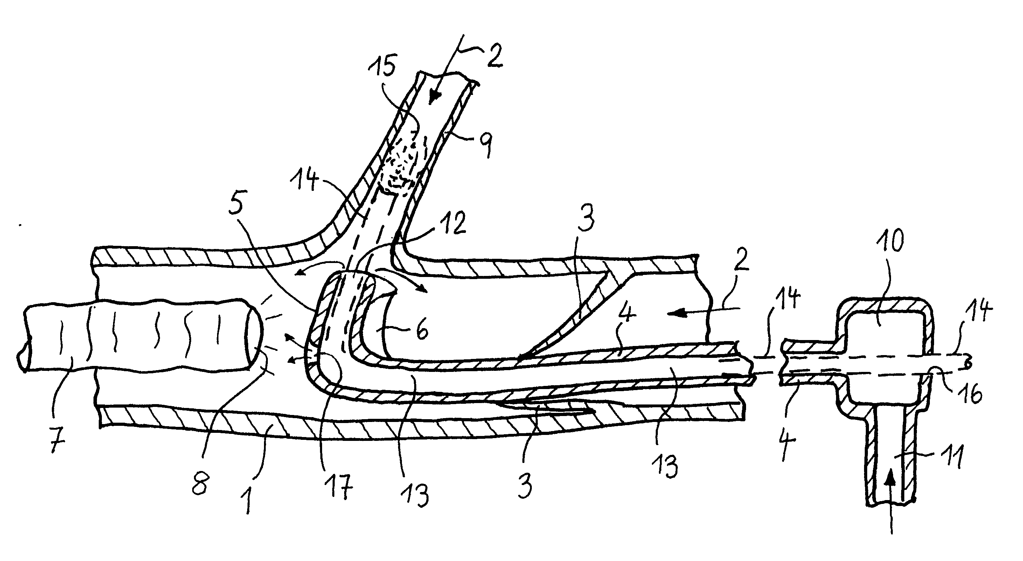

In der Zeichnung ist die Erfindung beispielsweise und schematisch im Schnitt durch ein Venenstück mit erfindungsgemäßem Messer und Endoskop dargestellt.

Die Figur zeigt ein Venenstück 1, das an seinen beiden nicht dargestellten Enden vom Blutkreislauf abgetrennt und nach außen geöffnet ist. Die ursprüngliche venöse Blutstromrichtung ist mit Pfeil 2 angedeutet.

Im dargestellten Venenstück 1 ist eine Venenklappe 3 dargestellt, die im wesentlichen aus den beiden dargestellten Klappenhälften besteht, die sich in Blutstromrichtung 2 öffnen und in Gegenrichtung schließen, also wie ein Rückschlagventil wirken.

Dieses Venenstück soll als Arterienbypass angesetzt werden. Das Blut soll also zukünftig durch dieses Venenstück in umgekehrter Richtung, also umgekehrt zur Richtung des Pfeiles 2 fließen. Die Venenklappe 3 würde dann dauernd schließen und muß daher aufgeschnitten werden.

Dazu dient das dargestellte Messer, das einen Schaft 4 und eine verrundete Klinge 5 mit nach hinten weisender Schneide 6 aufweist. Das Messer wird in Richtung des Pfeiles 2 vom einen Ende des Venenstückes her eingeführt, wobei es mit seiner verrundeten Klinge 5 ohne Verletzungsgefahr alle dazwischenliegenden Venenklappen durchdringt, ohne sie zunächst zu zerstören. Dann wird das Messer über den ganzen Weg zurückgezogen, wobei die nach rückwärts gerichtete Schneide 6 alle auf dem Wege liegenden Klappen aufschneidet, so daß ihre Rückschlagsperrwirkung beseitigt wird. Dieser Vorgang kann mehrmals unter verschiedenen Winkelstellungen der radial vom Schaft 4 abstehenden Klinge 5 durchgeführt werden.

Wird diese Arbeit blind durchgeführt, so kann es zu Problemen und unbefriedigenden Ergebnissen kommen. Daher wird in der Regel unter Zuhilfenahme eines Endoskopes 7 gearbeitet, üblicherweise eines flexiblen Endoskopes, das mit seinem Objektiv 8 der Längsbewegung des Messers entsprechend ständig nachgeführt wird und den Arbeitsbereich der Klinge 5 beobachten kann, wie in der Figur dargestellt. Es kann also durch das Objektiv 8 beobachtet werden, wie die Klinge 5 mit ihrer Schneide 6 die dargestellte Klappe zerschneidet. Auf diese Weise können die Resultate erheblich verbessert werden.

Das Sichtgebiet ist aber blutgefüllt. Es muß daher mit klarer Flüssigkeit gespült werden, um ein freies Sichtfeld zu schaffen, da Blut auch auf kurze Entfernungen völlig undurchsichtig ist.

Würde nur von den Enden des Venenstückes 1 her gespült werden, so würde sich gerade im dargestellten Venenabschnitt wenig ändern, da dort durch die Zweigvene 9 ständig Blut zufließt. Ein üblicher Ausweg wäre die Vorsehung eines Spülkanales am Endoskop 7. Das würde aber den Durchmesser dieses Instrumentes so weit vergrößern, daß eine Verwendung in der dargestellten kleinen Vene mit einem Innendurchmesser von typischerweise etwa 2 mm nicht mehr möglich wäre.

Es wird daher ein Arbeitskanal am Schaft 4 des Messers vorgesehen, der gleichzeitig als Spülkanal genutzt wird.

Ein Arbeits- und Spülkanal kann am Messerschaft auf vielfältige Weise vorgesehen werden. Es kann beispielsweise der Schaft in üblicher Weise als Draht ausgebildet sein, neben dem ein Schlauch oder Rohr vorgesehen ist.

Im dargestellten bevorzugten Ausführungsbeispiel ist der Schaft 4 als Rohr, also mit innenliegendem Arbeits- und Spülkanal 13, ausgebildet. Dieses Rohr kann flexibel als Kunststoffschlauch ausgebildet sein oder auch als starres Metallrohr, was bei langen geraden Venenstücken geometrisch möglich ist und den Vorteil eines besonders gut handhabbaren Messers schafft.

Am dargestellten rohrförmigen Schaft 4 ist am distalen Ende die Klinge 5 angeordnet. Am proximalen Ende ist ein Endstück 10 vorgesehen mit einem Spülzuflußschlauch 11, durch den in Pfeilrichtung Spülflüssigkeit zugeführt wird, die an der Austrittsöffnung 12 des Arbeits- und Spülkanales 13 ausströmt, was dort mit Pfeilen angedeutet ist. Die Spülflüssigkeit, in der Regel physiologische Kochsalzlösung, spült dort im Bereich des Sichtfeldes zwischen Objektiv 8 und Messer 5 und schafft klare Sicht.

Im dargestellten Ausführungsbeispiel verläuft der Arbeits- und und Spülkanal 13 zunächst durch den Schaft und sodann in gebogenem Verlauf durch die Klinge 5, um an deren vom Schaft abgelegenem freien Ende mit der Austrittsöffnung 12 zu enden. Die Austrittsöffnung 12 ist daher seitlich auf die Venenwand gerichtet, im dargestellten Fall auf den Mündungsbereich der Zweigvene 9.

Nun kann eine gestrichelt dargestellte flexible Sonde 14 durch den Arbeits- und Spülkanal 13 geführt werden. Unter Führung durch das Messer und unter Beobachtung durch das Endoskop kann die Sonde 14 mit ihrem distalen Ende bis in die Zweigvene 9 hinein geschoben werden, um dort beispielsweise einen Pfropfen aus Kunststoffmaterial 15 zu injizieren, der die Zweigvene 9 verschließt. Das ist für den späteren Betrieb als Arterie wesentlich, um Kurzschlüsse zwischen dem arteriellen und venösen Blutsystem zu verhindern.

An dem Endstück 10 am proximalen Ende des Messers ist eine Öffnung 16 vorgesehen, die beispielsweise mit flexiblen elastischen Gummilippen ausgebildet ist und die Sonde 14 derart elastisch abdichtend aufnimmt, daß auch bei vorgeschobener Sonde noch laufend weitergespült werden kann.

Im dargestellten Ausführungsbeispiel folgt der Arbeits- und Spülkanal 13 der Abbiegung der Klinge 5 und endet seitlich mit der Austrittsöffnung 12. In abgewandelten Ausführungsformen könnte der Spülkanal auch in axialer Verlängerung des Schaftes eine zweite Ausströmöffnung 17 aufweisen, um die Ausströmverhältnisse in erforderlicher Weise zu beeinflussen. Bei Bedarf können Ausströmöffnungen in anderer Weise angeordnet sein.

**Ansprüche**

1.  Messer zum Aufschneiden von Venenklappen mit einem langen, dünnen, einen Kanal aufweisenden Schaft und einer an dessen distalem Ende radial abstehend angeordneten Klinge mit rückwärts gerichteter Schneide, dadurch gekennzeichnet, daß der Kanal als am distalen Ende mit gekrümmtem Übergang seitlich abgewinkelter Arbeitskanal (13) ausgebildet ist.

2. Messer nach Anspruch 1, dadurch gekennzeichnet, daß der Schaft (4) rohrförmig als Arbeitskanal (13) ausgebildet ist.

3. Messer nach einem der vorhergehenden Ansprüche, dadurch gekennzeichnet, daß die Austrittsöffnung (12) des Arbeitskanals (13) am freien Ende der Klinge (5) angeordnet ist.

4. Messer nach einem der vorhergehenden Ansprüche, dadurch gekennzeichnet, daß der Arbeitskanal (13) gleichzeitig als Spülkanal ausgebildet ist.

5. Messer nach Anspruch 4, dadurch gekennzeichnet, daß der Arbeitskanal (13) zusätzliche Ausströmöffnungen (17) im distalen Endbereich des Schaftes aufweist.

**Europäisches**
**Patentamt**

# EUROPÄISCHER
# RECHERCHENBERICHT

Nummer der Anmeldung

# EP 90 12 3708

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | US-A-1 621 212   (MYERS)<br>* Seite 1, Zeilen 33-56; Figur 2 *<br>– – – | 1,2 | A 61 B<br>17/32<br>A 61 B 17/22 |
| A,D | WO-A-8 906 936   (MEDICAL INNOVATION)<br>* Seite 5, Zeilen 19-36; Figur 1 *<br>– – – – – | 1,2 | |

| RECHERCHIERTE SACHGEBIETE (Int. Cl.5) |
|---|
| A 61 B |

**Der vorliegende Recherchenbericht wurde für alle Patentansprüche erstellt**

| Recherchenort | Abschlußdatum der Recherche | Prüfer |
|---|---|---|
| Den Haag | 27 März 91 | MOERS R.J. |